# EUROPÄISCHE PATENTSCHRIFT

(11) **EP 3 628 221 B1**
(45) Veröffentlichungstag und Bekanntmachung des Hinweises auf die Patenterteilung: **07.07.2021**
(21) Anmeldenummer: 19401034.4
(22) Anmeldetag: 17.09.2019
(51) Int. Cl.: A61B 5/053, A61B 5/00, A61M 16/00

(54) **VORRICHTUNG ZUR ERFASSUNG DER LUNGENVENTILATION**
LUNG VENTILATION DETECTING DEVICE
DISPOSITIF DE DÉTECTION DE LA VENTILATION PULMONAIRE

(30) Priorität: 26.09.2018 DE 102018007602
(43) Veröffentlichungstag der Anmeldung: 01.04.2020
(73) Patentinhaber: Löwenstein Medical Technology S.A., 2557 Luxembourg (LU)
(72) Erfinder: Kremeier, Peter, 76149 Karlsruhe (DE)
(74) Vertreter: Marx, Thomas

(56) Entgegenhaltungen:
- DE-A1- 19 716 166
- DE-A1-102013 203 177
- DE-A1-102016 107 603
- DE-B3- 10 301 202
- US-A1- 2008 295 839

## Beschreibung

Bei einer Ateminsuffizienz führt die verminderte Ventilation der Lunge zu einer verminderten Sauerstoffaufnahme und in einigen Fällen auch zu einem erhöhten Kohlendioxid-Gehalt im Gewebe. Dies führt zur Veränderung der Blutgaswerte. Grundsätzlich sind Störungen der Oxygenierung von Störungen der Ventilation zu unterscheiden. Bei Störungen der Oxygenierung kann der Sauerstoffpartialdruck im Blut absinken, während der Körper den Kohlendioxidpartialdruck noch kompensieren kann. Bei Störungen der Ventilation können Sauerstoffpartialdruck und Kohlendioxidpartialdruck verändert sein.
Eine Methode zur Bestimmung der Ventilation der Lunge ist die Elektrischen Impedanz Tomographie (EIT). Bei der Elektrischen Impedanz Tomographie (EIT) fließen schwache Wechselströme auf Wegen des geringsten Widerstandes durch den Körper und erzeugen dabei elektrische Spannungen an der Oberfläche. Die gemessenen Oberflächenspannungen hängen von der Impedanz-(Widerstands)verteilung innerhalb des Thorax ab. Der gemessene Widerstand wird zum überwiegenden Teil durch den intrapulmonalen Luftgehalt bestimmt. Aufgrund der hohen zeitlichen Auflösung bietet das Verfahren die Möglichkeit, schnelle physiologische Veränderungen zu erfassen. Beispielsweise nutzt das Beatmungsgerät elisa 800 VIT (SALVIA medical GmbH & Co. KG, Niederhöchstädter Str. 62, 61476 Kronberg, Deutschland) die EIT-Technologie.
Unter Verwendung der EIT-Technologie lassen sich wenig belüftete Lungenareale identifizieren.
Gemäß dem Stand derTechnik können EIT-Daten vom Arzt genutzt werden, um die Ventilation der Lunge zu beurteilen.
Die Auswirkungen einer Veränderung der künstlichen Ventilation auf die Sauerstoffversorgung und den Kohlendioxidgehalt, insbesondere den Sauerstoffpartialdruck und den Kohlendioxidpartialdruck, werden jedoch bei der Einstellung der Beatmung klinisch nicht ausreichend berücksichtigt.

Die US 2008/0295839 A1 zeigt ein Beatmungssystem mit einer Sensoranordnung zur Überwachung der Kohlendioxid- und Sauerstoffwerte eines Patienten. Die aufgenommenen Werte dienen dabei der Steuerung des Beatmungsgerätes, insbesondere der Einstellung des PEEP.

Die DE 197 16 166 A1 beschreibt eine Vorrichtung zur Unterstützung der Atmung. Diese Vorrichtung ist unter anderem mit EIT-Sensoren ausgestattet, welche die Herzfunktion abhängig von der Beatmung überwachen.

Die DE 10 2016 107 603 A1 schlägt ein System zur Anzeige verschiedener Patientendaten auf einem Benutzerinterface vor. Dazu wird insbesondere auf verschiedene Darstellungsmöglichkeiten von EIT-Messungen eingegangen.

Die DE 103 01202 B3 zeigt eine Kombination aus Beatmungsgerät und EIT-Messsystem. Beide Geräte, Beatmungsgerät und EIT-Messsystem, sind dabei so ausgelegt, dass diese für einen bidirektionalen Datenaustausch ausgestattet sind.

Aus der DE 10 2013 203 177 A1 ist ein Beatmungsgerät zu entnehmen, welches auf der Basis von EIT-Messwerten automatisch gesteuert werden kann. Dabei wird insbesondere die Auswertung der EIT-Pixel diskutiert.

Aufgabe der Erfindung ist daher eine Beatmungsvorrichtung zu schaffen, bei der Informationen über die Ventilation der Lunge und Informationen über die Sauerstoffversorgung und den Kohlendioxidgehalt zur, beispielsweise automatischen, Steuerung oder Regelung der Beatmung genutzt werden.

Die Aufgabe wird gelöst durch eine:
Beatmungsvorrichtung, umfassend wenigstens ein Beatmungsgerät mit wenigstens einer steuerbaren Atemgasquelle und einer programmierbaren Steuereinrichtung und zumindest einer Sensoreinrichtung zur Bestimmung von Druck und/oder Fluss des Atemgases, wobei die Steuereinrichtung die Atemgasquelle zur Vorgabe eines ersten Beatmungsmusters (hinsichtlich Druck, Fluss, Volumen, Frequenz) ansteuert, wobei die Beatmungsvorrichtung wenigstens zwei weitere Sensoreinrichtungen aufweist wobei eine Sensoreinrichtung eine Vielzahl einzelner Sensoren aufweist die zur Erzeugung und/oder Messung von elektrischen Potenzialen ausgebildet sind und zur nichtinvasiven Bestimmung der elektrischen Impedanz (EI) der Lunge eines Patienten ausgebildet sind, wobei die zweite Sensoreinrichtung ausgebildet ist zur nichtinvasiven Bestimmung der Kohlendioxidversorgung, wobei die dritte Sensoreinrichtung ausgebildet ist zur nichtinvasiven Bestimmung der Sauerstoffversorgung des Patienten dadurch gekennzeichnet, dass die Steuereinrichtung die Sensormesswerte der EI-Sensoreinrichtung auswertet zur Bestimmung der momentanen Ventilation der Lunge während der Beatmung mit dem ersten Beatmungsmuster, wobei die Steuereinrichtung die Sensormesswerte der zweiten Sensoreinrichtung zur Bestimmung der momentanen Kohlendioxidversorgung auswertet und wobei die Steuereinrichtung die Sensormesswerte der dritten Sensoreinrichtung zur Bestimmung der momentanen Sauerstoffversorgung auswertet wobei die Steuereinrichtung die, anhand der Sensormessewerte ermittelten, Informationen über die Ventilation der Lunge und über die Sauerstoffversorgung und über die Kohlendioxidversorgung zur Steuerung oder Regelung der Beatmung nutzt.

Die Beatmungsvorrichtung ist auch dadurch gekennzeichnet, dass die Steuereinrichtung die Atemgasquelle zur Vorgabe eines ersten Beatmungsmusters ansteuert und die resultierenden ersten Sensor(mess)werte zumindest einer Sensoreinrichtung speichert, wobei die Steuereinrichtung die Atemgasquelle dann zur Vorgabe eines zweiten Beatmungsmusters ansteuert und die resultierenden zweiten Sensor(mess)werte zumindest einer Sensoreinrichtung speichert und die ersten Sensor(mess)werte mit den zweiten Sensormesswerten vergleicht.

Die Beatmungsvorrichtung ist auch dadurch gekennzeichnet, dass die Beatmungsvorrichtung zumindest eine mit der Steuereinrichtung kommunizierende Speichereinheit aufweist, in der zumindest Normwerte und/oder Grenzwerte für die Kohlendioxidversorgung und für die Sauerstoffversorgung hinterlegt sind.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensormesswerte der momentanen Kohlendioxidversorgung und/oder die Sensormesswerte der momentanen Sauerstoffversorgung von den Normwerten abweichen und/oder Grenzwerte zumindest erreicht sind.

Die Beatmungsvorrichtung ist auch dadurch gekennzeichnet, dass im Speicher für die Ventilation der Lunge Normwerte und Grenzwerte hinterlegt sind und die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die momentane Ventilation von den Normwerten abweicht und Grenzwerte zumindest erreicht sind.

Die Beatmungsvorrichtung ist auch dadurch gekennzeichnet, dass die Steuereinrichtung die Sensormesswerte der EI-Sensoreinrichtung auswertet zur Bestimmung der resultierenden Ventilation der Lunge während der Beatmung mit dem zweiten Beatmungsmuster, wobei die Steuereinrichtung die Sensormesswerte der zweiten Sensoreinrichtung auswertet zur Bestimmung der resultierenden Kohlendioxidversorgung während der Beatmung mit dem zweiten Beatmungsmuster , wobei die Steuereinrichtung die Sensormesswerte der dritten Sensoreinrichtung auswertet zur Bestimmung der resultierenden Sauerstoffversorgung während der Beatmung mit dem zweiten Beatmungsmuster und wobei die Steuereinrichtung die momentane Ventilation mit der resultierenden Ventilation vergleicht.

Die Beatmungsvorrichtung ist auch dadurch gekennzeichnet, dass die Steuereinrichtung die Atemgasquelle zur Vorgabe eines zweiten Beatmungsmusters derart ansteuert, dass die resultierende Ventilation der Lunge gegenüber der momentanen Ventilation verbessert ist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, sodass die Sensormesswerte der resultierenden Kohlendioxid- oder Sauerstoffversorgung sich Zielwerten zumindest annähern.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensormesswerte der momentanen Ventilation und/oder die Sensormesswerte des momentanen Sauerstoffpartialdruckes von den Normwerten abweichen und Grenzwerte zumindest erreicht sind.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein unverändertes EIT-Summensignal und eine hohe oder erhöhte EIT-Änderungsfrequenz identifiziert, wobei das zweite Beatmungsmuster eine, im Vergleich zum ersten Beatmungsmuster, erhöhte mandatorische Frequenz aufweist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein unverändertes EIT-Summensignal, im Vergleich zum ersten Beatmungsmuster, und ein erniedrigtes Maß für die Kohlendioxidversorgung aufweist, wobei das das zweite Beatmungsmuster eine, im Vergleich zum ersten Beatmungsmuster, erhöhte mandatorische Frequenz aufweist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein erniedrigtes EIT-Summensignal, im Vergleich zum ersten Beatmungsmuster, und ein erniedrigtes Maß für die Kohlendioxidversorgung aufweist, wobei das das zweite Beatmungsmuster eine, im Vergleich zum ersten Beatmungsmuster, erhöhte mandatorische Frequenz und/oder ein erhöhtes Tidalvolumen und/oder eine erhöhte Druckunterstützung aufweist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein unverändertes EIT-Summensignal und ein erhöhtes Maß für die Kohlendioxidversorgung aufweist, wobei das zweite Beatmungsmuster einen, im Vergleich zum ersten Beatmungsmuster, veränderten EPAP-Druck oder-Verlauf aufweist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein unverändertes EIT-Summensignal, im Vergleich zum ersten Beatmungsmuster , und ein erhöhtes Maß für die Kohlendioxidversorgung aufweist, wobei das das zweite Beatmungsmuster eine, im Vergleich zum ersten Beatmungsmuster, erhöhte mandatorische Frequenz aufweisen kann, wenn die EIT-Änderungsfrequenz erniedrigt ist.

Die Beatmungsvorrichtung ist auch dadurch gekennzeichnet, dass die Steuereinrichtung die Atemgasquelle zur Vorgabe eines zweiten Beatmungsmusters ansteuert, welches hinsichtlich Druck und/oder Fluss und/oder Volumen und/oder Frequenz von dem ersten Beatmungsmuster verschieden ist, wenn die Sensoreinrichtung ein erniedrigtes EIT-Summensignal, im Vergleich zum ersten Beatmungsmuster , und eine erniedrigte EIT-Änderungsfrequenz und ein erhöhtes Maß für die Kohlendioxidversorgung aufweist, wobei das das zweite Beatmungsmuster eine, im Vergleich zum ersten Beatmungsmuster, erhöhte mandatorische Frequenz und/oder ein erhöhtes Tidalvolumen und/oder eine erhöhte Druckunterstützung aufweist.

Die Beatmungsvorrichtung ist auch dadurch gekennzeichnet, dass die Steuereinrichtung die Atemgasquelle zur Vorgabe eines zweiten Beatmungsmusters ansteuert, wenn die Sensoreinrichtung ein erhöhtes EIT-Summensignal und/oder eine erniedrigte EIT-Änderungsfrequenz, im Vergleich zum ersten Beatmungsmuster, und ein erniedrigtes Maß für die Sauerstoffversorgung aufweist, wobei das zweite Beatmungsmuster eine, im Vergleich zum ersten Beatmungsmuster, erhöhte Frequenz aufweisen kann und/oder eine zumindest kurzzeitige Erhöhung der Inspirationszeit und/oder eine Anpassung des PEEP und/oder ein Wechsel des Beatmungsmusters.

Die Beatmungsvorrichtung ist auch dadurch gekennzeichnet, dass die Steuereinrichtung die Atemgasquelle zur Vorgabe eines ersten PEEP ansteuert und die resultierenden ersten Sensor(mess)werte zumindest einer Sensoreinrichtung speichert, wobei die Steuereinrichtung dann die Atemgasquelle zur Vorgabe eines zweiten PEEP ansteuert und die resultierenden zweiten Sensor(mess)werte zumindest einer Sensoreinrichtung speichert, wobei die Steuereinrichtung dann die ersten Sensor(mess)werte mit den zweiten Sensormesswerten vergleicht.

Die Beatmungsvorrichtung ist auch dadurch gekennzeichnet, dass die Steuereinrichtung die Atemgasquelle zur Vorgabe eines ersten Beatmungsmusters ansteuert, bei der der EPAP-Druck einen ersten Wert oder einen ersten Verlauf aufweist, wobei die Sensoreinrichtung zur nichtinvasiven Bestimmung der elektrischen Impedanz (EI) die resultierende Ventilation der Lunge bestimmt und wobei die zweite Sensoreinrichtung die Kohlendioxidversorgung bestimmt und/ oder wobei die dritte Sensoreinrichtung die Sauerstoffversorgung des Patienten bestimmt, wobei die Steuereinrichtung die Sensormesswerte speichert, die sich bei der Beatmung mit dem ersten Muster einstellen, wobei die Steuereinrichtung die Atemgasquelle zur Vorgabe eines zweiten Beatmungsmusters ansteuert, bei der der EPAP-Druck einen zweiten Wert oder einen zweiten Verlauf aufweist, wobei die Sensoreinrichtung zur nichtinvasiven Bestimmung der elektrischen Impedanz (EI) die resultierende Ventilation der Lunge bestimmt und wobei die zweite Sensoreinrichtung die Kohlendioxidversorgung bestimmt und/ oder wobei die dritte Sensoreinrichtung die Sauerstoffversorgung des Patienten bestimmt, wobei die Steuereinrichtung die Sensormesswerte speichert, die sich bei der Beatmung mit dem zweiten Muster einstellen, und wobei die Steuereinrichtung die Sensormesswerte die sich nach Applikation des ersten Beatmungsmusters einstellen mit den Sensormesswerte die sich nach Applikation des zweiten Beatmungsmusters einstellen vergleicht und/oder vergleichend auf dem Display darstellt.

Die Beatmungsvorrichtung ist auch dadurch gekennzeichnet, dass die Steuereinrichtung wenigstens ein Sensorsignal auf Fehler überwacht dadurch, dass bei Erreichen von Grenzwerten eines momentanen Sensorsignals dieses mit einem anderen momentanen Sensorsignal verglichen wird um eine Fehlerhaftigkeit zu erkennen.

Die Beatmungsvorrichtung ist auch dadurch gekennzeichnet, dass die Steuereinrichtung ein EIT-Sensorsignal auf Fehler überwacht dadurch, dass die Steuereinrichtung das EIT-Sensorsignal mit dem Sensorsignal einer Sensoreinrichtung zur Bestimmung von Druck und/oder Fluss des Atemgases zumindest zeitweise vergleicht.

Die Beatmungsvorrichtung ist auch dadurch gekennzeichnet, dass die Steuereinrichtung ein EIT-Sensorsignal auf Fehler überwacht dadurch, dass die Steuereinrichtung das EIT-Sensorsignal mit einem modellierten Sensorsignal vergleicht.

Die Beatmungsvorrichtung ist auch dadurch gekennzeichnet, dass das modellierten Sensorsignal im Wesentlichen kontinuierlich gelernt wird.

Sensoreinrichtung zur Verwendung mit einer Beatmungsvorrichtung nach zumindest einem der vorhergehenden Ansprüche.

Die erfindungsgemäße Vorrichtung hilft, frühzeitig Beatmungskomplikationen zu erkennen und zu vermeiden. Folgekosten werden dadurch verhindert.
Kostenaufwändige und komplikationsbehaftete innerklinische Transporte von beatmungspflichtigen Intensivpatienten werden reduziert.
Zu jedem Zeitpunkt ist eine differenzierte Aussage zur Ventilation der Lunge und zur Versorgung mit Sauerstoff und/oder Kohlendioxid möglich.
Eine differenzierte Beurteilung der Ventilation war bisher nur als Momentaufnahme mit bildgebenden Verfahren (z.B. Thorax-CT) möglich. Mit der neuen Technologie kann die Ventilation kontinuierlich analysiert und überwacht werden. Durch die Einblendung der Lungenkonturen des Patienten können räumliche Ventilationsverteilungen zugeordnet und therapeutisch bewertet werden. Die Darstellung erfolgt nichtinvasiv, hochauflösend und in Echtzeit.

Mit der erfindungsgemäßen Vorrichtung kann die Situation der Lunge in Echtzeit beurteilt und die Auswirkungen der Anpassung der Beatmungseinstellungen fortlaufend beurteilt werden. Atemzyklische Kollapsareale und Überdehnungsbezirke sind identifizierbar und können bewertet werden.

Weitere Vorteile und Merkmale der vorliegenden Erfindung ergeben sich aus der Beschreibung der Ausführungsbeispiele, welche mit Bezug auf die beiliegende Figur im Folgenden erläutert werden.

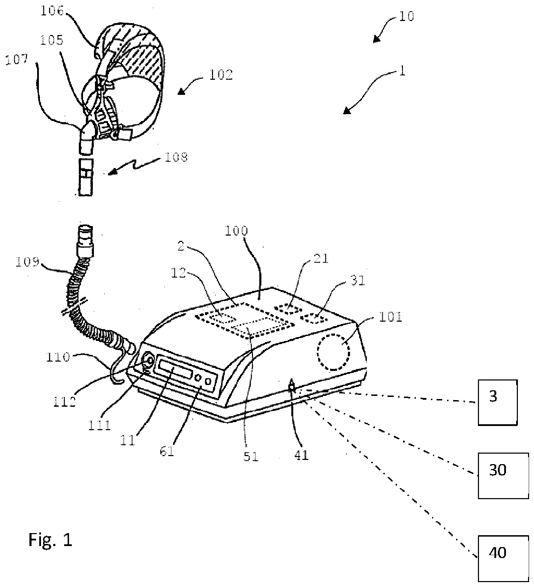

In der Figur 1 ist eine erfindungsgemäße Beatmungsvorrichtung 10 gezeigt, welche hier ein als Heimbeatmungsgerät oder Schlaftherapiegerät oder klinisches Beatmungsgerät ausgeführt ist. Das Beatmungsgerät 1 und die Beatmungsvorrichtung 10 sind zur Durchführung des erfindungsgemäßen Verfahrens ausgebildet.
Die Beatmungsvorrichtung (10) umfasst wenigstens ein Beatmungsgerät (1) mit wenigstens einer steuerbaren Atemgasquelle (100) und einer programmierbaren Steuereinrichtung (21) und zumindest einer Sensoreinrichtung (2) zur Bestimmung von Druck und/oder Fluss des Atemgases, wobei die Steuereinrichtung (21) die Atemgasquelle zur Vorgabe eines ersten vorbestimmten Beatmungsmusters (45) (hinsichtlich Druck, Fluss, Volumen, Frequenz) ansteuert. Die Beatmungsvorrichtung (10) umfasst zudem wenigstens zwei weitere räumlich von dem Beatmungsgerät (1) getrennte Sensoreinrichtungen (3, 30, 40), die mit der Steuereinrichtung (21) der Beatmungsvorrichtung (10) kommunizieren. Ein Sensor (3) weist eine Vielzahl einzelner Sensoreinrichtungen (3, 3', 3" ...) auf und jede Sensoreinrichtung ist zur Erzeugung von elektrischen Potenzialen und/oder zur nichtinvasiven Bestimmung der elektrischen Impedanz (EI) eines Körperabschnitts und zur Ermittlung und Übertragung der Sensormesswerte an die Steuereinheit ausgebildet. Die programmierbare Steuereinrichtung (21) wertet die Sensormesswerte der EI-Sensoreinrichtung (3) aus, zur Bestimmung der momentanen Ventilation (44) der Lunge während der Beatmung mit dem ersten Beatmungsmuster (45). Die Steuereinrichtung (21) steuert die Atemgasquelle beispielsweise auch zur Vorgabe eines zweiten Beatmungsmusters (55) an, welches hinsichtlich Druck und/oder Fluss und/oder Volumen und/oder Frequenz von dem ersten Beatmungsmuster (45) verschieden ist, wobei die Steuereinrichtung (21) die Sensormesswerte der EI-Sensoreinrichtung (3) auswertet zur Bestimmung der resultierenden Ventilation (54) der Lunge während der Beatmung mit dem zweiten Beatmungsmuster (55), wobei die Steuereinrichtung (21) die momentanen Ventilation (44) mit der resultierenden Ventilation (54) vergleicht zur Feststellung des besser geeigneten Beatmungsmusters. Wobei ein Anwender Vorgaben machen kann, um Zielwerte vorzugeben, die erreicht werden müssen um ein "besser geeignetes Beatmungsmuster" zu erreichen. Wobei alternativ auch oder ergänzend Änderungen der Kohlendioxid- und/oder Sauerstoff-Meßwerte ein wichtiges Kriterium sind, die bei der Auswahl oder der Fortführung von Beatmungsmustern entscheidungserheblich sind.
Das Beatmungsgerät 1 umfasst eine Atemgasquelle 100 mit beispielsweise einer Gebläseeinrichtung und/oder einer Ventileinrichtung 101 zur Erzeugung eines Luftstromes für die Beatmung. Zur Steuerung der Atemgasquelle 100 und zur Erfassung und Verarbeitung von Sensordaten ist hier eine Steuereinrichtung 21 vorgesehen. Die Bedienung und Einstellung des Beatmungsgerätes 1 erfolgt über eine Benutzerschnittstelle 61 mit Bedienelementen und einer Anzeigeeinrichtung 11 (Display).
Das Beatmungsgerät 1 weist eine Atemschnittstelle 102 auf, um den Luftstrom einem Patienten zur Beatmung zuzuführen. Die hier gezeigte Atemschnittstelle 102 ist eine beispielhaft als Nasalmaske ausgebildete Atemmaske 105. Zur Fixierung der Atemmaske 105 ist beispielsweise eine Kopfhaube 106 vorgesehen. Die Atemschnittstelle 102 kann beispielsweise auch als eine Vollgesichtsmaske, als eine Nasenbrille, als ein Tubus oder als eine Larynxmaske ausgestaltet sein.
Zur Verbindung der Atemschnittstelle 102 mit der Atemgasquelle 100 ist zumindest ein Verbindungsschlauch 109 vorgesehen, der über eine Koppeleinrichtung 112 luftführend mit der Atemgasquelle 100 verbunden wird. Über ein Kopplungselement 107 wird der Verbindungsschlauch 109 mit der Atemschnittstelle 102 verbunden. Zwischen dem Verbindungsschlauch 109 und dem Kopplungselement 107 ist ein Ausatmungselement 108 angeordnet, welches ein Ventil umfasst oder als ein solches ausgebildet ist. Das Ausatmungselement 108 ist beispielsweise dazu vorgesehen, während der Ausatmung des Benutzers ein Rückatmen in das Beatmungsgerät 1 zu verhindern.
Die Steuereinrichtung 21 ist mit einer nicht näher dargestellten Sensoreinrichtung (2,3,30,40) verbunden, welche einen oder mehrere Sensoren zur Erfassung von Geräteparametern und/oder Patientenparametern und/oder anderer für die Beatmung charakteristischer Größen aufweist. Die Steuereinrichtung 21 kann als eine zentrale Steuereinrichtung, beispielsweise im dem Beatmungsgerät, ausgeführt sein, die alle Sensorwerte verarbeitet. Die Steuereinrichtung 21 kann in Form mehrerer dezentraler Steuereinrichtungen, beispielsweise eine im dem Beatmungsgerät und (je) eine, die einem (oder den) Sensor(en) zugeordnet ist, ausgeführt sein. für die Beatmung charakteristischer Größen aufweist.

Zum Beispiel umfasst die Steuereinrichtung 21 einen hier nicht näher gezeigten Drucksensor (2) welcher die Druckverhältnisse des Atemgases ermittelt. Dazu ist der Drucksensor, beispielsweise über einen Druckmessschlauch 110 zu der Atemschnittstelle 102, mit dem Atemgas wirkverbunden. Über einen Eingangsstutzen 111 ist der Druckmessschlauch 110 an die Steuereinrichtung 21 angebunden. Zum Beispiel umfasst die Steuereinrichtung 21 auch einen hier nicht näher gezeigten Flusssensor (2), welcher die Flussverhältnisse des Atemgases ermittelt.
Des Weiteren dient die Steuereinrichtung 21 hier zur Ansteuerung der Gebläseeinrichtung und/oder der Ventileinrichtung 101. Die Steuereinrichtung 21 stellt einen notwendigen Minimaldruck bereit und kompensiert Druckschwankungen, die durch die Atemtätigkeit des Benutzers bedingt sind. Beispielsweise erfasst die Steuereinrichtung 21 auch den gegenwärtigen Druck in der Atemmaske 105 (oder dem Patienteninterface) und regelt die Leistung der Atemgasquelle entsprechend nach, bis ein gewünschter Beatmungsdruck anliegt. Die zur Einstellung der Atemgasquelle 100 benötigten Geräteparameter sowie die Gerätekonfiguration und/oder Gerätesoftware sind in einer Speichereinrichtung 31 hinterlegt.

Die Steuereinrichtung 21 kann auch den Sauerstoffgehalt im Atemgas regeln, durch entsprechende Ansteuerung der Gebläseeinrichtung und/oder der Ventileinrichtung 101, wobei die Ventileinrichtung zumindest eine Sauerstoffquelle (Druckgasflasche oder Krankenhausleitung) aufweist oder konnektiert. Die Sauerstoffzumischung zu dem Atemgasstrom erfolgt beispielsweise stromabwärts oder stromaufwärts von der Gebläseeinrichtung. In diesem Fall ist die Ventileinrichtung ein Sauerstoffmischventil. Die Sauerstoffzumischung zu dem Atemgasstrom erfolgt beispielsweise durch eine Ventileinheit, die den Atemgasstrom und den Sauerstoffstrom führt und regelt.
Die Steuereinrichtung 21 kann auch zur Erfassung von sensorisch erfassten Patientenparametern ausgebildet sein. Die Steuereinrichtung 21 kann dazu mit Sensoren zur Messung der Atemexkursion, zur Messung einer Sauerstoffversorgung und/oder einer Kohlendioxidversorgung und /oder zur Messung einer EEG-, einer EMG-, einer EOG- oder einer EKG-Aktivität ausgestattet sein.
Die Beatmungsvorrichtung weist insbesondere wenigstens zwei räumlich von dem Beatmungsgerät (1) getrennte, Sensoreinrichtungen (3,30,40) auf, die mit der Steuereinheit (21) kommunizieren, wobei ein Sensor (3) eine Vielzahl einzelner Sensoreinrichtungen (3, 3', 3" ...) aufweist und jede Sensoreinrichtung zur Erzeugung von elektrischen Potenzialen ausgebildet ist (13) zur nichtinvasiven Bestimmung der elektrischen Impedanz (EI) eines Körperabschnitts. Die Sensoreinrichtungen (3) sind beispielsweise als Klebepflaster ausgeführt, mit einer der Haut abgewandten Außenschicht und einer der Haut zugewandten adhäsiven Schicht, oder als Gurt zusammengefasst, der die Sensoreinrichtungen haltert.

Die Beatmungsvorrichtung (10) umfasst zumindest eine weitere Sensoreinrichtung (30), die zur nichtinvasiven Bestimmung des CO2 (30) ausgebildet ist. Die Sensoreinrichtung (30) ist beispielsweise als Klemmsensor oder als Klebepflaster ausgeführt mit einer der Haut abgewandten Außenschicht und einer der Haut zugewandten adhäsiven Schicht. Die Beatmungsvorrichtung (10) umfasst zumindest eine weitere Sensoreinrichtung (40), die zur nichtinvasiven Bestimmung des O2 (40) ausgebildet ist. Die Sensoreinrichtung (30) ist beispielsweise als Klemmsensor oder als Klebepflaster ausgeführt mit einer der Haut abgewandten Außenschicht und einer der Haut zugewandten adhäsiven Schicht.

Die Sensoren (3, 30,40) umfassen beispielsweise Energie- und Sendemittelmittel zur Ermittlung und kabellosen Übertragung der Sensormesswerte. Die Sensoren (3, 30,40) umfassen alternativ beispielsweise Kommunikationsmittel (35, 45) zur Übertragung der Sensormesswerte an die Steuerung (21).

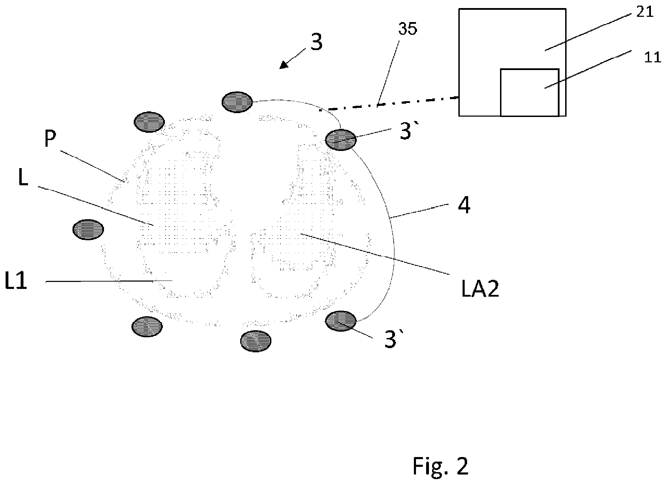

Fig. 2 zeigt eine schematische Darstellung der EIT-Messung.
Die Sensoreinrichtung (3) weist eine Vielzahl einzelner Sensoren (3, 3', 3" ...) auf, die zur Erzeugung und/oder Messung von elektrischen Potenzialen ausgebildet sind und zur nichtinvasiven Bestimmung der elektrischen Impedanz (EI) der Lunge (L) eines Patienten (P) dienen.

Die Sensoreinrichtung weist beispielsweise 32 hochauflösende Sensoren (3,3'...) in einem textilen Gürtel, nicht dargestellt, auf. Ein, nicht dargestellter, integrierter Lagesensor bestimmt die Position des Patienten. Die Sensoren sind durch Kommunikations- oder Leitungsmittel (4) miteinander verbunden (4) und so angeordnet, dass sie Signale nacheinander aus allen Richtungen empfangen können. Die Steuereinheit (21) kann die Sensormesswerte der EIT-Messung (3) daher räumlich zuordnen. Damit können die lokalen Gewebswiderstände aus unterschiedlichen Richtungen ermittelt und dann in bewegte Bilder umgerechnet werden. Die Steuereinheit (21) verarbeitet die Sensormesswerte der EIT-Messung (3) derart, dass für einzelne Lungenabschnitte (LA1, LA2) eine spezifische elektrische Impedanz (EI) ermittelt wird. Die Sensoreinrichtung (3) ist über kabelgebundene oder drahtlose Kommunikationsmittel (35) mit der Steuereinheit (21) verbunden. Die Steuereinheit (21) speichert (11) und verarbeitet die Sensormesswerte zur Verwendung in der Beatmungsvorrichtung. Der Speichereinrichtung (31) kann Bestandteil der Steuereinheit (21) sein oder ein separater Baustein.
Fig. 2 zeigt auch eine schematische Darstellung der Ergebnisse der EIT-Messung auf dem Display der Beatmungsvorrichtung. Die Steuereinheit (21) verarbeitet die Sensormesswerte zur Verwendung in der Beatmungsvorrichtung derart, dass auf dem Display der Beatmungsvorrichtung (10) eine Visualisierung des Patiententhorax (P) mit den beiden Lungenflügeln (L) dargestellt wird. Die Steuereinheit (21) verarbeitet die Sensormesswerte der EIT-Messung (3) derart, dass für einzelne Lungenabschnitte (LA1, LA2) eine spezifische elektrische Impedanz (EI) der Lunge (L) ermittelt wird. Die spezifische elektrische Impedanz (EI) der Lungenabschnitte (LA) wird für die Darstellung am Display derart aufbereitet, dass Lungenabschnitte mit einer hohen elektrischen Impedanz (L2) graphisch anders dargestellt werden, als solche Lungenabschnitte mit einer niedrigen elektrischen Impedanz (L1). Die Steuereinheit (21) verarbeitet die Sensormesswerte der EIT-Messung (3) zeitlich derart, dass für einzelne Lungenabschnitte (LA1, LA2) eine spezifische elektrische Impedanz (EI) der Lunge (L) atemzugweise ermittelt wird und für die Darstellung am Display aufbereitet wird.
Die Sensoreinrichtung (3) ist (zusammen mit der Steuerung und dem Speicher) auch eingerichtet und ausgebildet, ein EIT-Summensignal (44') zu bilden, welches ein Maß für die Ventilation der Lunge bzw. von Lungenabschnitten ist. Die Sensoreinrichtung (3) ist auch (zusammen mit der Steuerung und dem Speicher) eingerichtet und ausgebildet, eine Häufigkeit der EIT-Änderungsfrequenz (44") zu ermitteln. Die Sensoreinrichtung (3) kann somit zusammenfassend die momentane Ventilation (44) ermitteln.
Im Sinne der Erfindung wird unter dem EIT-Summensignal (44') verstanden, dass die Impedanz- oder Impedanzänderungsverteilung in der durch die Sensoren bestimmten Schnittebene oder dem durch die Sensoren umspannten Volumen nicht zwingend für die Durchführung des erfindungsgemäßen Verfahrens bildlich beispielsweise auf einem Display dargestellt werden muss, zumindest aber die berechneten Ergebniswerte der Impedanzverteilung oder Impedanzänderungsverteilung (für die Durchführung des Verfahrens) in der erfindungsgemäßen Vorrichtung vorliegen.
Bei einer Summation sämtlicher Impedanzwerte der Sensoren ergibt sich demnach bei der Inspiration ein geändertes Summensignal im Vergleich zu dem Summensignal bei der Exspiration. Der Summenwert stellt demnach ein adäquates Maß dar, um die Ventilation der Lunge zu bestimmen.
Die EIT-Änderungsfrequenz (44") lässt sich aus der atemphasenabhängigen Änderung der Impedanzwerte der Sensoren bestimmen. Die Impedanzwerte der Sensoren schwanken atemphasenabhängig mit der Inspiration und der Exspiration. Die EIT-Änderungsfrequenz (44") stellt demnach ein alternatives oder ergänzendes Maß dar, um die (Frequenz der) Ventilation der Lunge zu bestimmen.

Beispielsweise sind die Sensoren in einen textilen und atmungsaktiven und dehnbaren Elektrodengürtel, zusammen mit einem Lagesensor, integriert. Der Sensor (3) und die Steuereinheit sind eingerichtet eine Bildrate von beispielsweise bis zu 50 pro Sekunde zu erzeugen. Dabei werden Lungenbereiche (LA) visualisiert. Erfindungsgemäß sind patientenbezogene Eingaben möglich, wie Körpergröße, Körpergewicht, Geschlecht, Brustkorbumfang.
Die so ermittelten und aufbereiteten EIT-Daten ermöglichen eine zeitlich und räumlich aufgelöste differenzierte Analyse und Darstellung der elektrischen Impedanz (EI) der Lunge.

Erfindungsgemäß wird die Mess- und Regelungstechnik der Beatmungsvorrichtung mit der Elektrischen Impedanz Tomographie (EIT) vernetzt. Dadurch kann die Funktion der Lunge kontinuierlich bildgebend dargestellt werden. Die Messwerte der Beatmungsvorrichtung werden mit den Ergebnissen der elektrischen Impedanztomographie kombiniert. So ermöglicht es diese Technologie, die vielfältigsten klinischen Fragestellungen zu bewerten und die Therapie entsprechend auszurichten.

Die Gase Kohlendioxid (CO₂) und Sauerstoff (O₂) können leicht durch Körpergewebe und auch durch die Haut diffundieren. Nicht-invasive Sensoren, der auf der Haut anliegen, können O2 und CO2 messen, wenn die Haut neben dem Sensor auf eine Temperatur, die über der Körpertemperatur liegt, erwärmt wird. Durch die Wärme, typisch 42° bis 45°C, erhöht sich der lokale Kapillarblutfluss und die Gasdiffusion wird gesteigert. Dies erlaubt eine recht präzise Bestimmung der CO₂/O₂-Messwerte an der Hautoberfläche.

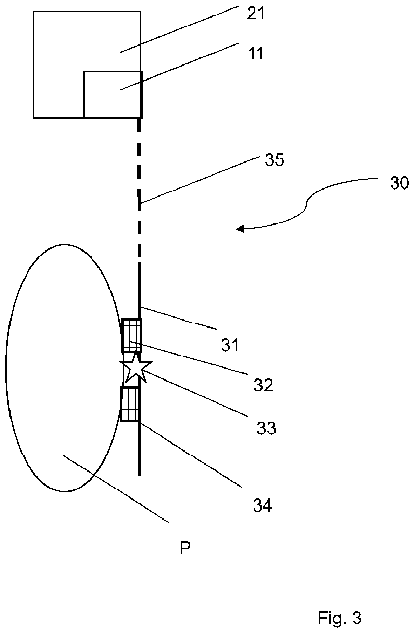

Fig. 3 zeigt eine schematische Darstellung dertranskutanen Blutgasanalyse von tpCO2. Mithilfe der transkutanen Kohlendioxidmessung kann nicht invasiv die Kohlendioxidspannung der Haut (tpCO2) bestimmt werden. Die Messgenauigkeit hängt weniger stark von der Durchblutung ab, als die tpO2. Der pCO2 an der Hautoberfläche liegt bei 44°C etwa 1,37-fach über dem arteriellen pCO2. Der erfindungsgemäß verwendete Sensor berücksichtigt diesen Zusammenhang durch einen Korrekturfaktor. Der pCO₂-Sensor erwärmt die Haut typisch auf 42° bis 45°C und verwendet eine pH-Glaselektrode in einer dünnen Elektrolytschicht unter einer hydrophoben, CO₂- und O₂-durchlässigen Membran. Eine Auswerteeinheit wandelt die Signale der Interaktion an der Elektrode in ein elektrisches Signal um und sendet dieses an die Steuereinheit.

Die transkutane Blutgasmessung ermöglicht die Messung von tpCO2 durch die Haut eines Patienten. Die grundlegende Funktionsweise des Sensors wird in Abbildung 3 dargestellt. Das Kohlendioxid diffundiert aus der Haut des Patienten (P) in den Sensor (32, 34). Der Sensor weist zumindest ein Sensorelement auf (32, 34) welches sensitiv für Kohlendioxid ist; es können auch zwei oder mehr Sensorelemente (32, 34) verwendet werden. Eine Wärmequelle (33) sorgt für eine Erwärmung (42-45 °C) der Haut des Patienten (P), die dem Sensor benachbart ist. Die erhöhte Temperatur führt zu einer gesteigerten Durchblutung der Haut und des Kapillarbettes. Dadurch wird vermehrt Kohlendioxid freigesetzt. Der Sensor verfügt über eine Ansprechzeit im Sekundenbereich bis Minutenbereich. Der Sensor ermöglicht einen stabilen Betrieb von mindestens 5 h ohne Signaldrift. Der Sensor (30) ist über kabelgebundene oder drahtlose Kommunikationsmittel (35) mit der Steuereinheit (21) verbunden. Die Steuereinheit (21) speichert (11) und verarbeitet die Sensormesswerte zur Verwendung in der Beatmungsvorrichtung. Der Speicher (31) kann Bestandteil der Steuereinheit (21) sein oder ein separater Baustein.
Die Sensoreinrichtung (30) ist (zusammen mit der Steuereinheit (21) und dem Speicher) eingerichtet und ausgebildet, ein momentanes Maß (46) für die Kohlendioxidversorgung, beispielsweise den Kohlendioxidpartialdruck, zu bilden.

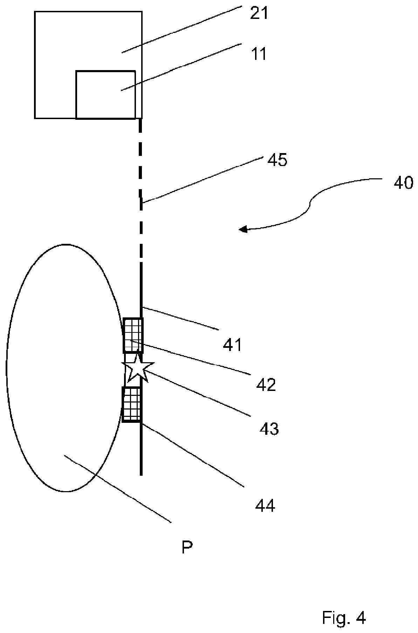

Fig. 4 zeigt eine schematische Darstellung der transkutanen Blutgasanalyse von pO2. Mithilfe der transkutanen Sauerstoffmessung kann nicht invasiv die Sauerstoffspannung der Haut (tpO2) bestimmt werden. Die Messgenauigkeit hängt von der Durchblutung ab. Wird im gleichen arteriellen Versorgungsgebiet gemessen, besteht eine enge Korrelation zwischen tpO2 und der arteriellen Sauerstoff pO2. Ein beispielhafter Sensor beinhaltet fluoreszierende Farbstoffe in der Sensor-Oberfläche. Die Sauerstoffmoleküle interagieren mit den fluoreszierenden Farbstoffen. Eine Auswerteeinheit wandelt die Signale der Interaktion in ein elektrisches Signal um und sendet dieses an die Steuereinheit.

Die transkutane Blutgasmessung ermöglicht die Messung von pO2 durch die Haut eines Patienten. Die grundlegende Funktionsweise des Sensors wird in Fig. 4 dargestellt. Sauerstoff diffundiert aus der Haut des Patienten (P) in den Sensor (42, 44). Der Sensor weist zumindest ein Sensorelement auf (42, 44) welches sensitiv für Sauerstoff ist; es können auch zwei oder mehr Sensorelemente verwendet werden. Eine Wärmequelle (43) sorgt für eine Erwärmung (42-45°C) der Haut des Patienten (P), die dem Sensor benachbart ist. Die erhöhte Temperatur führt zu einer gesteigerten Durchblutung der Haut und des Kapillarbettes. Dadurch wird vermehrt O2 freigesetzt. Der Sensor verfügt über eine Ansprechzeit im Sekundenbereich bis Minutenbereich. Der Sensor ermöglicht einen stabilen Betrieb von mindestens 5 h ohne Signaldrift. Der Sensor (40) ist über kabelgebundene oder drahtlose Kommunikationsmittel (45) mit der Steuereinheit (21) verbunden. Die Steuereinheit (21) speichert (11) und verarbeitet die Sensormesswerte zur Verwendung in der Beatmungsvorrichtung. Der Speicher (31) kann Bestandteil der Steuereinheit (21) sein oder ein separater Baustein. Die Pulsoximetrie ist eine weitere nicht invasive Methode zur Messung der arteriellen Sauerstoffsättigung des Blutes. Etablierte Systeme verwenden zwei Wellenlängen zur Differenzierung zwischen oxygeniertem und desoxygeniertem Blut. Die Signale werden erfasst, indem rotes und infrarotes Licht durch ein Kapillarbett (Finger oder Ohrläppchen) geleitet wird und während des Pulszyklus die Änderungen der Lichtabsorption gemessen werden. Der Sensor verwendet rote und infrarote Leuchtdioden (LEDs), die Licht durch das Kapillarbett zu einer Photodiode senden. Die Photodiode empfängt das Licht, wandelt es in ein elektrisches Signal um und sendet dieses zur Berechnung an die Auswerteeinheit oder die Steuereinheit. Die Sensoreinrichtung (40) ist (zusammen mit der Steuereinheit (21) und dem Speicher) eingerichtet und ausgebildet, ein momentanes Maß (47) für die Sauerstoffversorgung, beispielsweise den Sauerstoffpartialdruck, zu bilden.

Die Tabellen 1 und 2 zeigen wie die Steuereinrichtung (21) die Atemgasquelle zur Vorgabe eines Beatmungsmusters (45, 55) ansteuert, und wir die Steuereinrichtung (21) zudem Sensormesswerte der momentanen Kohlendioxidversorgung (46) und/oder die Sensormesswerte des momentanen (47) Sauerstoffversorgung für die Vorgabe eines Beatmungsmusters (45, 55) berücksichtigt. Die Vorgabe eines Beatmungsmusters (45, 55) kann dabei unmittelbar erfolgen, beispielsweise in Form von Steuerbefehlen für die Atemgasquelle. Die Vorgabe eines Beatmungsmusters (45, 55) kann dabei mittelbar erfolgen, beispielsweise in Form eines Beatmungsmusters (45, 55) welches dem Betreuer oder Bediener der Beatmungsvorrichtung (beispielsweise am Display) vorgeschlagen wird.

Die Tabellen 1 und 2 zeigen wie die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensormesswerte der momentanen Kohlendioxidversorgung (46) und/oder die Sensormesswerte des momentanen (47) Sauerstoffversorgung von den Normwerten (62) abweichen.

**Tabelle 1**

| EIT-Summensignal (44') relative Signale Impedanz 32 Elektroden überwachen sich selbst, bis zu 7 Elektroden können fehlen | Häufigkeit der EIT-Änderungsfrequenz (=Atemfrequenz) (44") | momentanes Maß (46) Kohlendioxidpartialdruck Normwert Partialdruck 35 - 45 mmHg Grenzwert unter 35 oder über 50 | Vorgabe (55) Steuereinheit (21) an Beatmungsgerät (1) |
|---|---|---|---|
| unverändert | Hoch | Niedrig | Erhöhung der mandatorischen Frequenz |
| niedrig | Normal | Niedrig | Erhöhung Tidalvolumen, Delta-P oder Druckunterstützung Erhöhung der mandatorischen Frequenz |
| Unverändert | Niedrig | Hoch | Erhöhung der mandatorischen Frequenz |
| Niedrig | Niedrig | hoch | Erhöhung Tidalvolumen, Delta-P oder Druckunterstützung Erhöhung der mandatorischen Frequenz |
| Hoch | Unverändert | Hoch | Reduzierung des Tidalvolumen oder Delta-P Automatische Manöver zur Beurteilung der endinspiratorischen Überdehnung |
| Unverändert | Unverändert | Hoch | Beatmung fortführen Ursachensuche (extrapulmonaler Grund) |

Die Tabellen 1 und 2 zeigen ebenfalls wie die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensormesswerte der momentanen Ventilation (44) und/oder die Sensormesswerte des momentanen Kohlendioxidpartialdruckes (46) von den Normwerten abweichen und Grenzwerte zumindest erreicht sind.

**Tabelle 2**

| EIT-Summensignal (44') | Häufigkeit der EIT-Änderungsfrequenz (=Atemfrequenz) (44") | momentanes Maß (47) Sauerstoffpartialdruck Normwert Partialdruck 75 - 100 mmHg Grenzwert unter 70 oder über 105 | Vorgabe Steuereinheit (21) an Beatmungsgerät (1) |
|---|---|---|---|
| niedrig | Niedrig | niedrig | Kurzzeitige Erhöhung o2 Ggfs. im Verlauf wieder o2 reduzieren Erhöhung Tidalvolumen, Delta-P oder Druckunterstützung Erhöhung der mandatorischen Frequenz Wechsel des Beatmungsmusters |
| Unverändert | Unverändert | niedrig | Kurzzeitige Erhöhung o2 PEEP-Anpassungen |
| Hoch | Niedrig | niedrig | Erhöhung Frequenz Erhöhung Inspirationszeit Wechsel des Beatmungsmusters |

Die Tabellen 1 und 2 zeigen auch wie die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensormesswerte der momentanen Ventilation (44) und/oder die Sensormesswerte des momentanen (47) Sauerstoffpartialdruckes von den Normwerten (62) abweichen und Grenzwerte (63) zumindest erreicht sind.

Die Tabellen 3 zeigt, wie die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, damit die Sensormesswerte des resultierenden Kohlendioxidpartialdruckes oder Sauerstoffpartialdruckes Zielwerte erreichen.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein unverändertes EIT-Summensignal (44') und eine hohe oder erhöhte EIT-Änderungsfrequenz (44"), wobei das zweite Beatmungsmuster (55) eine, im Vergleich zum ersten Beatmungsmuster (45), erhöhte mandatorische Frequenz aufweisen kann.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein unverändertes EIT-Summensignal (44'), im Vergleich zum ersten Beatmungsmuster (45), und ein erniedrigtes Maß für die Kohlendioxidversorgung aufweist, wobei das das zweite Beatmungsmuster (55) eine, im Vergleich zum ersten Beatmungsmuster (45), erhöhte mandatorische Frequenz aufweisen kann.

Die Steuereinrichtung (21) steuert die Atemgasquelle zur Vorgabe eines zweiten Beatmungsmusters (55) an, wenn die Sensoreinrichtung (3) ein erniedrigtes EIT-Summensignal (44'), im Vergleich zum ersten Beatmungsmuster (45), und ein erniedrigtes Maß für die Kohlendioxidversorgung aufweist, wobei das das zweite Beatmungsmuster (55) eine, im Vergleich zum ersten Beatmungsmuster (45), erhöhte mandatorische Frequenz und/oder ein erhöhtes Tidalvolumen und/oder eine erhöhte Druckunterstützung aufweisen kann.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein unverändertes EIT-Summensignal (44') und ein erhöhtes Maß für die Kohlendioxidversorgung aufweist, wobei das zweite Beatmungsmuster (55) einen, im Vergleich zum ersten Beatmungsmuster (45), veränderten EPAP-Druck oder-Verlauf aufweist. Der EPAP-Druck kann in diesem Sinne auch oder einen ersten Verlauf aufweisen, der in nachfolgenden Zyklen der Beatmung automatisch oder auf Anwenderauswahl in geändert wird.

Beispielsweise kann der EPAP zu Beginn der Ausatmung ansteigen und dann abfallen oder zur Mitte der Ausatmung einen Maximalwert oder Minimalwert einnehmen. Alternativ kann der EPAP auch zum Ende der Ausatmung einen Maximalwert einnehmen. Der EPAP kann auch während der Ausatmung unterschiedliche Druckniveaus annehmen. Auch kann der EPAP über einen längeren Zeitraum appliziert werden.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein unverändertes EIT-Summensignal (44'), im Vergleich zum ersten Beatmungsmuster (45), und ein erhöhtes Maß für die Kohlendioxidversorgung aufweist, wobei das das zweite Beatmungsmuster (55) eine, im Vergleich zum ersten Beatmungsmuster (45), erhöhte mandatorische Frequenz aufweisen kann, wenn die EIT-Änderungsfrequenz (44") erniedrigt ist.

Die Steuereinrichtung (21) steuert die Atemgasquelle zur Vorgabe eines zweiten Beatmungsmusters (55) an, wenn die Sensoreinrichtung (3) ein erniedrigtes EIT-Summensignal (44'), im Vergleich zum ersten Beatmungsmuster (45), und eine erniedrigte EIT-Änderungsfrequenz (44") und ein erhöhtes Maß für die Kohlendioxidversorgung aufweist, wobei das das zweite Beatmungsmuster (55) eine, im Vergleich zum ersten Beatmungsmuster (45), erhöhte mandatorische Frequenz und/oder ein erhöhtes Tidalvolumen und/oder eine erhöhte Druckunterstützung aufweisen kann.

Die Steuereinrichtung (21) steuert die Atemgasquelle zur Vorgabe eines zweiten Beatmungsmusters (55) an, wenn die Sensoreinrichtung (3) ein erhöhtes EIT-Summensignal (44'), im Vergleich zum ersten Beatmungsmuster (45), und eine unveränderte EIT-Änderungsfrequenz (44") und ein erhöhtes Maß für die Kohlendioxidversorgung aufweist, wobei das das zweite Beatmungsmuster (55) eine, im Vergleich zum ersten Beatmungsmuster (45), ein reduziertes Tidalvolumen und/oder ein Automatisches Manöver zur Beurteilung der endinspiratorischen Überdehnung aufweisen kann.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein erniedrigtes EIT-Summensignal (44') und/oder eine erniedrigte EIT-Änderungsfrequenz, im Vergleich zum ersten Beatmungsmuster (45), und ein erniedrigtes Maß für die Sauerstoffversorgung aufweist, wobei das das zweite Beatmungsmuster (55) eine, im Vergleich zum ersten Beatmungsmuster (45), erhöhte mandatorische Frequenz aufweisen kann und/oder eine zumindest kurzzeitige Erhöhung der Sauerstoffbeimischung zum Atemgas und/oder eine Erhöhung von Tidalvolumen, Delta-P oder Druckunterstützung und/oder ein Wechsel des Beatmungsmusters.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein unverändertes EIT-Summensignal (44') und/oder eine unveränderte EIT-Änderungsfrequenz, im Vergleich zum ersten Beatmungsmuster (45), und ein erniedrigtes Maß für die Sauerstoffversorgung aufweist, wobei das das zweite Beatmungsmuster (55) eine, im Vergleich zum ersten Beatmungsmuster (45), erhöhte mandatorische Frequenz aufweisen kann und/oder eine zumindest kurzzeitige Erhöhung der Sauerstoffbeimischung zum Atemgas und/oder eine Anpassung des PEEP.

Die Steuereinrichtung (21) steuert die Atemgasquelle zur Vorgabe eines zweiten Beatmungsmusters (55) (hinsichtlich Druck, Fluss, Volumen, Frequenz) an, wenn die Sensoreinrichtung (3) ein erhöhtes EIT-Summensignal (44') und/oder eine erniedrigte EIT-Änderungsfrequenz, im Vergleich zum ersten Beatmungsmuster (45), und ein erniedrigtes Maß für die Sauerstoffversorgung aufweist, wobei das zweite Beatmungsmuster (55) eine, im Vergleich zum ersten Beatmungsmuster (45), erhöhte Frequenz aufweisen kann und/oder eine zumindest kurzzeitige Erhöhung der Inspirationszeit und/oder eine Anpassung des PEEP und/oder ein Wechsel des Beatmungsmusters.

**Tabelle 3**

| Beatmungsgerät angesteuert | Sensorisches Monitoring resultierender Kohlendioxid und/oder Sauerstoff und/oder EIT |
|---|---|
| (schrittweise) Verstellen des PEEPS (zur Austestung des individuell notwendigen PEEPS) | Solange bis Sauerstoff erhöht oder stabil bleibt, ohne dass das Kohlendioxid sich erhöht |
| (schrittweise) Senken der inspiratorische Sauerstoffkonzentration | Solange bis Sauerstoff den gewünschten Zielkorridor erreicht |
| (schrittweise) Verstellen des IPAP und/oder EPAP | Solange bis Sauerstoff erhöht oder stabil bleibt, ohne dass das Kohlendioxid sich erhöht und/oder EIT-Summensignal unverändert |
| (schrittweise) Verstellen der Frequenz | Solange bis Sauerstoff erhöht oder stabil bleibt, ohne dass das Kohlendioxid sich erhöht und/oder EIT-Summensignal unverändert |

Tabelle 3 ist erfindungsgemäß so zu verstehen: Die Steuereinrichtung (21) steuert die Atemgasquelle zur Vorgabe eines ersten PEEP (45) an und speichert die resultierenden ersten Sensor(mess)werte zumindest einer Sensoreinrichtung (2,3,30,40). Die Steuereinrichtung (21) steuert die Atemgasquelle zur Vorgabe eines zweiten PEEP (55) an und speichert die resultierenden zweiten Sensor(mess)werte zumindest einer Sensoreinrichtung (2,3,30,40). Die Steuereinrichtung (21) vergleicht dann die ersten Sensor(mess)werte mit den zweiten Sensormesswerten zur Ermittlung des besser geeigneten PEEP-Druckes.
Diese Vorgehensweisen kann für n unterschiedliche PEEP-Drücke erfolgen zur Bestimmung des idealen PEEP-Druckes.

Tabelle 3 ist erfindungsgemäß generell so zu verstehen: Die Steuereinrichtung (21) steuert die Atemgasquelle zur Vorgabe eines ersten Beatmungsmusters (45) an und speichert die resultierenden ersten Sensor(mess)werte zumindest einer Sensoreinrichtung (2,3,30,40). Die Steuereinrichtung (21) steuert die Atemgasquelle zur Vorgabe eines zweiten Beatmungsmusters (55) an und speichert die resultierenden zweiten Sensor(mess)werte zumindest einer Sensoreinrichtung (2,3,30,40).
Die Steuereinrichtung (21) vergleicht dann die ersten Sensor(mess)werte mit den zweiten Sensormesswerten zur Ermittlung des besser geeigneten Beatmungsmusters.
Diese Vorgehensweisen kann für n unterschiedliche Beatmungsmuster erfolgen zur Bestimmung des idealen Beatmungsmusters (45,55 ...).
Die Entscheidung welches Beatmungsmuster besser geeignet oder optimal ist, wird dabei anhand hinterlegter oder hinterlegbarer Normwerte und Grenzwert für zumindest einen, bevorzugt zumindest zwei der Sensormesswerte beispielsweise als Vorschlag für den Arzt oder Anwender auf dem Display der Vorrichtung oder per Fernübertragung zu einem Display übermittelt. Der Anwender kann daraufhin das besser geeignete Beatmungsmuster anwählen und ausführen lassen oder das Beatmungsmuster noch weiter modifizieren und dann ausführen lassen. Alternativ kann die Vorrichtung selbst, anhand hinterlegter oder hinterlegbarer Normwerte und Grenzwert für zumindest einen, bevorzugt zumindest zwei der Sensormesswerte bestimmen, welches Beatmungsmuster besser geeignet ist und dieses ausführen.

Beispielsweise erfolgt durch die Steuereinrichtung 21 eine Regelung auf Soll-Geräteparameter des Beatmungsgerätes, welche zuvor anhand der charakteristischen Atmung eines Patienten, die sensorisch (2,3,30,40) ermittelt wurde, individuell berechnet und festgelegt worden sind. Es ist auch möglich, dass die Atemgasquelle 100 dynamisch und insbesondere je nach Atemphase des Patienten angepasst wird. Beispielsweise kann anhand der Steuereinrichtung 21 ein Atemphasenwechsel erkannt werden, sodass je nach Atemphase ein höherer bzw. niedrigerer Druck bereitgestellt werden kann. Beispielsweise reagiert das Beatmungsgerät auf bestimmte Atemereignisse, wie z. B. Schnarchen, Atemabflachungen und/oder obstruktive Druckspitzen mit entsprechenden Einstellungen der Geräteparameter.
Die von der Steuereinrichtung 21 erfassten Druckverhältnisse werden zusammen mit weiteren Geräteparametern in einer Speichereinrichtung 31 hinterlegt. Zudem werden die von der Steuereinrichtung 21 eingestellten Druckverhältnisse bzw. die vorgenommenen Druckanpassungen ebenfalls als Geräteparameter in der Speichereinrichtung 31 hinterlegt. Auch die erfassten Patientenparameter können in der der Speichereinrichtung 31 hinterlegt werden. Auch die Sensorwerte der Sensoren (2,3,30,40) werden in der Speichereinrichtung 31 zumindest teilweise abgelegt.
Als Geräteparameter können beispielsweise ein Start-Therapie-Druck, ein maximaler Therapie-Druck, ein minimaler Therapie-Druck und/oder ein Zielvolumen und/oder andere zur Einstellung der Atemgasquelle 100 geeignete Geräteparameter hinterlegt sein. Diese Geräteparameter werden von der Steuereinrichtung 21 zur Einstellung der Atemgasquelle 100 aus der Speichereinrichtung 31 abgerufen.

Des Weiteren werden die über den Therapiezeitraum erfassten Druckverhältnisse und/oder sonstigen Geräteparameter und/oder Patientenparameter im Rahmen von Therapieverläufen in der Speichereinrichtung 31 hinterlegt. Als Therapieverläufe können z. B. ein Fluss-Verlauf, ein Druck-Verlauf und/oder ein Event-Verlauf registriert werden. Die Therapieverläufe werden der Speichereinrichtung 31 von der Steuereinrichtung 21 bereitgestellt, die diese Daten während der Therapie erfasst.
Die Therapiedaten können auch über ein im Gerät 1 befindliches und/oder an das Gerät 1 angeschlossenes Display 11 bzw. eine Benutzerschnittstelle 61 ausgelesen werden.
In einigen Fällen (Ventilationsinsuffizienz, früher respiratorische Globalinsuffizienz) mit erhöhten pCO₂-Werten ist die Zufuhr von Sauerstoff und die Abatmung des Kohlendioxids nur mit gleichzeitiger Beatmung möglich.

Beispielsweise steuert die Steuereinrichtung (21) die Atemgasquelle zur Vorgabe eines ersten Beatmungsmusters (45) an bei der EPAP-Druck einen ersten Wert oder einen ersten Verlauf aufweist, wobei die Sensoreinrichtung (3) zur nichtinvasiven Bestimmung der elektrischen Impedanz (EI) die resultierende Ventilation der Lunge bestimmt und wobei die zweite Sensoreinrichtung (30) die Kohlendioxidversorgung bestimmt und/ oder wobei die dritte Sensoreinrichtung (40) die Sauerstoffversorgung des Patienten bestimmt. Die Steuereinrichtung (21) speichert die Sensormesswerte, die sich bei der Beatmung mit dem ersten Muster einstellen.

Der EPAP-Druck kann einen ersten Wert aufweisen, der in nachfolgenden Zyklen der Beatmung automatisch oder auf Anwenderauswahl in geändert wird. Die Änderung kann eine Erhöhung oder Erniedrigung sein. Das Ziel der Änderung ist jeweils den EPAP-Druck zu finden, bei dem die Sensormesswerte (3,30,40) als eine Verbesserung der Ventilation und/oder der Sauerstoffversorgung interpretiert werden können.

Der EPAP-Druck kann in diesem Sinne auch oder einen ersten Verlauf aufweisen, der in nachfolgenden Zyklen der Beatmung automatisch oder auf Anwenderauswahl in geändert wird.
Beispielsweise kann der EPAP zu Beginn der Ausatmung ansteigen und dann abfallen oder zur Mitte der Ausatmung einen Maximalwert oder Minimalwert einnehmen. Alternativ kann der EPAP auch zum Ende der Ausatmung einen Maximalwert einnehmen. Der EPAP kann auch während der Ausatmung unterschiedliche Druckniveaus annehmen.
Eine solche Titration kann auch für den IPAP (getrennt von dem EPAP oder in synchronisierter Änderung mit dem EPAP) vorgenommen werden. Der IPAP-Druck kann in diesem Sinne auch oder einen ersten Verlauf aufweisen, der in nachfolgenden Zyklen der Beatmung automatisch oder auf Anwenderauswahl in geändert wird.
Beispielsweise kann der IPAP zu Beginn der Einatmung ansteigen und dann abfallen oder zur Mitte der Einatmung einen Maximalwert oder Minimalwert einnehmen. Alternativ kann der IPAP auch zum Ende der Einatmung einen Maximalwert einnehmen.

## Patentansprüche

1. Beatmungsvorrichtung (10), umfassend wenigstens ein Beatmungsgerät (1) mit wenigstens einer steuerbaren Atemgasquelle (100) und einer programmierbaren Steuereinrichtung (21) und zumindest einer Sensoreinrichtung (2) zur Bestimmung von Druck und/oder Fluss des Atemgases, wobei die Steuereinrichtung (21) die Atemgasquelle zur Vorgabe eines ersten Beatmungsmusters (45) (hinsichtlich Druck, Fluss, Volumen, Frequenz) ansteuert, wobei die Beatmungsvorrichtung wenigstens zwei weitere Sensoreinrichtungen (3, 30, 40) aufweist wobei eine Sensoreinrichtung (3) eine Vielzahl einzelner Sensoren (3, 3', 3" ...) aufweist die zur Erzeugung und/oder Messung von elektrischen Potenzialen ausgebildet sind und zur nichtinvasiven Bestimmung der elektrischen Impedanz (EI) der Lunge eines Patienten ausgebildet sind, wobei die zweite Sensoreinrichtung (30) ausgebildet ist zur nichtinvasiven Bestimmung der Kohlendioxidversorgung, wobei die dritte Sensoreinrichtung (40) ausgebildet ist zur nichtinvasiven Bestimmung der Sauerstoffversorgung des Patienten wobei die Steuereinrichtung (21) die Sensormesswerte der EI-Sensoreinrichtung (3) auswertet zur Bestimmung der momentanen Ventilation (44) der Lunge während der Beatmung mit dem ersten Beatmungsmuster (45), wobei die Steuereinrichtung (21) die Sensormesswerte der zweiten Sensoreinrichtung (30) zur Bestimmung der momentanen Kohlendioxidversorgung (46) auswertet und wobei die Steuereinrichtung (21) die Sensormesswerte der dritten Sensoreinrichtung 40 zur Bestimmung der momentanen (47) Sauerstoffversorgung auswertet, **dadurch gekennzeichnet, dass** die Steuereinrichtung (21) die Sensormesswerte der EI-Sensoreinrichtung (3) auswertet zur Bestimmung der resultierenden Ventilation (54) der Lunge während der Beatmung mit dem zweiten Beatmungsmuster (55), wobei die Steuereinrichtung (21) die Sensormesswerte der zweiten Sensoreinrichtung (30) auswertet zur Bestimmung der resultierenden Kohlendioxidversorgung (56) während der Beatmung mit dem zweiten Beatmungsmuster (55), wobei die Steuereinrichtung (21) die Sensormesswerte der dritten Sensoreinrichtung (40) auswertet zur Bestimmung der resultierenden Sauerstoffversorgung (57) während der Beatmung mit dem zweiten Beatmungsmuster (55) und wobei die Steuereinrichtung (21) die momentane Ventilation (44) mit der resultierenden Ventilation (54) vergleicht, wobei die Steuereinrichtung (21) die, anhand der Sensormessewerte ermittelten, Informationen über die Ventilation der Lunge und über die Sauerstoffversorgung und über die Kohlendioxidversorgung zur Steuerung oder Regelung der Beatmung nutzt.

2. Beatmungsvorrichtung nach Anspruch 1 **dadurch gekennzeichnet, dass** die Steuereinrichtung (21) die Atemgasquelle zur Vorgabe eines ersten Beatmungsmusters (45) ansteuert und die resultierenden ersten Sensor(mess)werte zumindest einer Sensoreinrichtung (2,3,30,40) speichert, wobei die Steuereinrichtung (21) die Atemgasquelle dann zur Vorgabe eines zweiten Beatmungsmusters (55) ansteuert und die resultierenden zweiten Sensor(mess)werte zumindest einer Sensoreinrichtung (2,3,30,40) speichert und die ersten Sensor(mess)werte mit den zweiten Sensormesswerten vergleicht.

3. Beatmungsvorrichtung nach Anspruch 1 oder 2 **dadurch gekennzeichnet, dass** die Beatmungsvorrichtung (10) zumindest eine mit der Steuereinrichtung kommunizierende Speichereinheit (31) aufweist, in der zumindest Normwerte (62) und/oder Grenzwerte (63) für die Kohlendioxidversorgung und für die Sauerstoffversorgung hinterlegt sind.

4. Beatmungsvorrichtung nach Anspruch 1,2 oder 3 **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensormesswerte der momentanen Kohlendioxidversorgung (46) und/oder die Sensormesswerte der momentanen (47) Sauerstoffversorgung von den Normwerten (62) abweichen und/oder Grenzwerte (63) zumindest erreicht sind.

5. Beatmungsvorrichtung nach zumindest einem der vorhergehenden Ansprüche **dadurch gekennzeichnet, dass** im Speicher (31) für die Ventilation (44) der Lunge Normwerte (72) und Grenzwerte (73) hinterlegt sind und die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die momentane Ventilation (44) von den Normwerten (72) abweicht und Grenzwerte (73) zumindest erreicht sind.

6. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, sodass die Sensormesswerte der resultierenden Kohlendioxid- oder Sauerstoffversorgung sich Zielwerten zumindest annähern.

7. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensormesswerte der momentanen Ventilation (44) und/oder die Sensormesswerte des momentanen (47) Sauerstoffpartialdruckes von den Normwerten (62) abweichen und Grenzwerte (63) zumindest erreicht sind.

8. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensoreinrichtung (3) ein unverändertes EIT-Summensignal (44') und eine hohe oder erhöhte EIT-Änderungsfrequenz (44") identifiziert, wobei das zweite Beatmungsmuster (55) eine, im Vergleich zum ersten Beatmungsmuster (45), erhöhte mandatorische Frequenz aufweist.

9. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensoreinrichtung (3) ein unverändertes EIT-Summensignal (44'), im Vergleich zum ersten Beatmungsmuster (45), und ein erniedrigtes Maß für die Kohlendioxidversorgung aufweist, wobei das das zweite Beatmungsmuster (55) eine, im Vergleich zum ersten Beatmungsmuster (45), erhöhte mandatorische Frequenz aufweist.

10. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensoreinrichtung (3) ein erniedrigtes EIT-Summensignal (44'), im Vergleich zum ersten Beatmungsmuster (45), und ein erniedrigtes Maß für die Kohlendioxidversorgung aufweist, wobei das das zweite Beatmungsmuster (55) eine, im Vergleich zum ersten Beatmungsmuster (45), erhöhte mandatorische Frequenz und/oder ein erhöhtes Tidalvolumen und/oder eine erhöhte Druckunterstützung aufweist.

11. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensoreinrichtung (3) ein unverändertes EIT-Summensignal (44') und ein erhöhtes Maß für die Kohlendioxidversorgung aufweist, wobei das zweite Beatmungsmuster (55) einen, im Vergleich zum ersten Beatmungsmuster (45), veränderten EPAP-Druck oder-Verlauf aufweist.

12. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, welches hinsichtlich Druck und/oder Fluss und/oder Volumen und/oder Frequenz von dem ersten Beatmungsmuster (45) verschieden ist, wenn die Sensoreinrichtung (3) ein unverändertes EIT-Summensignal (44'), im Vergleich zum ersten Beatmungsmuster (45), und ein erhöhtes Maß für die Kohlendioxidversorgung aufweist, wobei das das zweite Beatmungsmuster (55) eine, im Vergleich zum ersten Beatmungsmuster (45), erhöhte mandatorische Frequenz aufweisen kann, wenn die EIT-Änderungsfrequenz (44") erniedrigt ist.

13. Beatmungsvorrichtung nach zumindest einem der vorhergehenden Ansprüche **dadurch gekennzeichnet, dass** die Steuereinrichtung (21) die Atemgasquelle zur Vorgabe eines zweiten Beatmungsmusters (55) ansteuert, wenn die Sensoreinrichtung (3) ein erhöhtes EIT-Summensignal (44') und/oder eine erniedrigte EIT-Änderungsfrequenz, im Vergleich zum ersten Beatmungsmuster (45), und ein erniedrigtes Maß für die Sauerstoffversorgung aufweist, wobei das zweite Beatmungsmuster (55) eine, im Vergleich zum ersten Beatmungsmuster (45), erhöhte Frequenz aufweisen kann und/oder eine zumindest kurzzeitige Erhöhung der Inspirationszeit und/oder eine Anpassung des PEEP und/oder ein Wechsel des Beatmungsmusters.

14. Beatmungsvorrichtung nach zumindest einem der vorhergehenden Ansprüche **dadurch gekennzeichnet, dass** die Steuereinrichtung (21) die Atemgasquelle zur Vorgabe eines ersten PEEP (45) ansteuert und die resultierenden ersten Sensor(mess)werte zumindest einer Sensoreinrichtung (2,3,30,40) speichert, wobei die Steuereinrichtung (21) dann die Atemgasquelle zur Vorgabe eines zweiten PEEP (55) ansteuert und die resultierenden zweiten Sensor(mess)werte zumindest einer Sensoreinrichtung (2,3,30,40) speichert, wobei die Steuereinrichtung (21) dann die ersten Sensor(mess)werte mit den zweiten Sensormesswerten vergleicht.

15. Beatmungsvorrichtung nach zumindest einem der vorhergehenden Ansprüche **dadurch gekennzeichnet, dass** die Steuereinrichtung (21) die Atemgasquelle zur Vorgabe eines ersten Beatmungsmusters (45) ansteuert, bei der der EPAP-Druck einen ersten Wert oder einen ersten Verlauf aufweist, wobei die Sensoreinrichtung (3) zur nichtinvasiven Bestimmung der elektrischen Impedanz (EI) die resultierende Ventilation der Lunge bestimmt und wobei die zweite Sensoreinrichtung (30) die Kohlendioxidversorgung bestimmt und/ oder wobei die dritte Sensoreinrichtung (40) die Sauerstoffversorgung des Patienten bestimmt, wobei die Steuereinrichtung (21) die Sensormesswerte speichert, die sich bei der Beatmung mit dem ersten Muster einstellen, wobei die Steuereinrichtung (21) die Atemgasquelle zur Vorgabe eines zweiten Beatmungsmusters (55) ansteuert, bei der der EPAP-Druck einen zweiten Wert oder einen zweiten Verlauf aufweist, wobei die Sensoreinrichtung (3) zur nichtinvasiven Bestimmung der elektrischen Impedanz (EI) die resultierende Ventilation der Lunge bestimmt und wobei die zweite Sensoreinrichtung (30) die Kohlendioxidversorgung bestimmt und/ oder wobei die dritte Sensoreinrichtung (40) die Sauerstoffversorgung des Patienten bestimmt, wobei die Steuereinrichtung (21) die Sensormesswerte speichert, die sich bei der Beatmung mit dem zweiten Muster einstellen, und wobei die Steuereinrichtung (21) die Sensormesswerte die sich nach Applikation des ersten Beatmungsmusters einstellen mit den Sensormesswerte die sich nach Applikation des zweiten Beatmungsmusters einstellen vergleicht und/oder vergleichend auf dem Display darstellt.

16. Beatmungsvorrichtung nach zumindest einem der vorhergehenden Ansprüche **dadurch gekennzeichnet, dass** die Steuereinrichtung (21) wenigstens ein Sensorsignal (2,3,30,40) auf Fehler überwacht dadurch, dass bei Erreichen von Grenzwerten eines momentanen Sensorsignals (2,3,30,40) dieses mit einem anderen momentanen Sensorsignal (2,3,30,40) verglichen wird um eine Fehlerhaftigkeit zu erkennen.

## Claims

1. An artificial respiration device (10) comprising at least one artificial respirator (1) having at least one controllable breathing gas source (100) and a programmable control apparatus (21) and at least one sensor apparatus (2) for determining the pressure and/or flow of the breathing gas, wherein the control apparatus (21) activates the breathing gas source for predetermining a first artificial respiration pattern (45) (relative to pressure, flow, volume, frequency), wherein the artificial respiration device has at least two further sensor apparatuses (3, 30, 40), wherein a sensor apparatus (3) has a plurality of individual sensors (3, 3', 3",...) which are configured for generating and/or measuring electrical potentials and which are configured for the non-invasive determination of the electrical impedance (EI) of the lungs of a patient, wherein the second sensor apparatus (30) is configured for the non-invasive determination of the carbon dioxide supply, wherein the third sensor apparatus (40) is configured for the non-invasive determination of the oxygen supply of the patient, wherein the control apparatus (21) evaluates the sensor measured values of the EI sensor apparatus (3) for determining the instantaneous ventilation (44) of the lungs during artificial respiration with the first artificial respiration pattern (45), wherein the control apparatus (21) evaluates the sensor measured values of the second sensor apparatus (30) for determining the instantaneous carbon dioxide supply (46) and wherein the control apparatus (21) evaluates the sensor measured values of the third sensor apparatus 40 for determining the instantaneous (47) oxygen supply, **characterized in that** the control apparatus (21) evaluates the sensor measured values of the EI sensor apparatus (3) for determining the resulting ventilation (54) of the lungs during artificial respiration with the second artificial respiration pattern (55), wherein the control apparatus (21) evaluates the sensor measured values of the second sensor apparatus (30) for determining the resulting carbon dioxide supply (56) during artificial respiration with the second artificial respiration pattern (55), wherein the control apparatus (21) evaluates the sensor measured values of the third sensor apparatus (40) for determining the resulting oxygen supply (57) during artificial respiration with the second artificial respiration pattern (55) and wherein the control apparatus (21) compares the instantaneous ventilation (44) with the resulting ventilation (54), wherein the control apparatus (21) uses the information about the ventilation of the lungs and about the oxygen supply and about the carbon dioxide supply, which is determined using the sensor measured values, for controlling or regulating the artificial respiration.

2. The artificial respiration device according to claim 1, **characterized in that** the control apparatus (21) activates the breathing gas source for predetermining a first artificial respiration pattern (45) and stores the resulting first sensor (measured) values of at least one sensor apparatus (2, 3, 30, 40), wherein the control apparatus (21) then activates the breathing gas source for predetermining a second artificial respiration pattern (55) and stores the resulting second sensor (measured) values of at least one sensor apparatus (2, 3, 30, 40) and compares the first sensor (measured) values with the second sensor measured values.

3. The artificial respiration device according to claim 1 or 2, **characterized in that** the artificial respiration device (10) has at least one memory unit (31) which communicates with the control apparatus and in which at least standard values (62) and/or limit values (63) for the carbon dioxide supply and for the oxygen supply are stored.

4. The artificial respiration device according to claim 1, 2 or 3, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor measured values of the instantaneous carbon dioxide supply (46) and/or the sensor measured values of the instantaneous (47) oxygen supply deviate from the standard values (62) and/or limit values (63) are at least reached.

5. The artificial respiration device according to at least one of the preceding claims, **characterized in that** standard values (72) and limit values (73) for the ventilation (44) of the lungs are stored in the memory (31) and the control apparatus (21) activates the breathing gas source for predetermining a second artificial respiration pattern (55), which is different from the first artificial respiration pattern (45) regarding pressure and/or flow and/or volume and/or frequency, when the instantaneous ventilation (44) deviates from the standard values (72) and limit values (73) are at least reached.

6. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, such that the sensor measured values of the resulting carbon dioxide supply or oxygen supply at least approach target values.

7. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor measured values of the instantaneous ventilation (44) and/or the sensor measured values of the instantaneous (47) oxygen partial pressure deviate from the standard values (62) and limit values (63) are at least reached.

8. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor apparatus (3) identifies an unchanged EIT sum signal (44') and a high or increased EIT change frequency (44"), wherein the second artificial respiration pattern (55) has an increased mandatory frequency in comparison with the first artificial respiration pattern (45).

9. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor apparatus (3) has an unchanged EIT sum signal (44') in comparison with the first artificial respiration pattern (45) and a reduced level of the carbon dioxide supply, wherein the second artificial respiration pattern (55) has an increased mandatory frequency in comparison with the first artificial respiration pattern (45).

10. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor apparatus (3) has a reduced EIT sum signal (44') in comparison with the first artificial respiration pattern (45) and a reduced level of the carbon dioxide supply, wherein the second artificial respiration pattern (55) has an increased mandatory frequency and/or an increased tidal volume and/or an increased pressure support in comparison with the first artificial respiration pattern (45).

11. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor apparatus (3) has an unchanged EIT sum signal (44') and an increased level of the carbon dioxide supply, wherein the second artificial respiration pattern (55) has a changed EPAP pressure or EPAP path in comparison with the first artificial respiration pattern (45).

12. The artificial respiration device according to at least one of the preceding claims, **characterized in that** the control apparatus (21) activates the breathing gas source for predetermining a second artificial respiration pattern (55), which is different from the first artificial respiration pattern (45) regarding pressure and/or flow and/or volume and/or frequency, when the sensor apparatus (3) has an unchanged EIT sum signal (44') in comparison with the first artificial respiration pattern (45) and an increased level of the carbon dioxide supply, wherein the second artificial respiration pattern (55) may have an increased mandatory frequency in comparison with the first artificial respiration pattern (45) when the EIT change frequency (44") is reduced.

13. The artificial respiration device according to at least one of the preceding claims, **characterized in that** the control apparatus (21) activates the breathing gas source for predetermining a second artificial respiration pattern (55) when the sensor apparatus (3) has an increased EIT sum signal (44') and/or a reduced EIT change frequency, in comparison with the first artificial respiration pattern (45), and a reduced level of the oxygen supply, wherein the second artificial respiration pattern (55) may have an increased frequency in comparison with the first artificial respiration pattern (45) and/or an at least temporary increase in the inspiration time and/or an adaptation of the PEEP and/or a change in the artificial respiration pattern.

14. The artificial respiration device according to at least one of the preceding claims, **characterized in that** the control apparatus (21) activates the breathing gas source for predetermining a first PEEP (45) and stores the resulting first sensor (measured) values of at least one sensor apparatus (2, 3, 30, 40), wherein the control apparatus (21) then activates the breathing gas source for predetermining a second PEEP (55) and stores the resulting second sensor (measured) values of at least one sensor apparatus (2, 3, 30, 40), wherein the control apparatus (21) then compares the first sensor (measured) values with the second sensor measured values.

15. The artificial respiration device according to at least one of the preceding claims, **characterized in that** the control apparatus (21) activates the breathing gas source for predetermining a first artificial respiration pattern (45) in which the EPAP pressure has a first value or a first path, wherein the sensor apparatus (3) for the non-invasive determination of the electrical impedance (EI) determines the resulting ventilation of the lungs and wherein the second sensor apparatus (30) determines the carbon dioxide supply and/or wherein the third sensor apparatus (40) determines the oxygen supply of the patient, wherein the control apparatus (21) stores the sensor measured values which are present during artificial respiration with the first pattern, and wherein the control apparatus (21) activates the breathing gas source for predetermining a second artificial respiration pattern (55) in which the EPAP pressure has a second value or a second path, wherein for the non-invasive determination of the electrical impedance (EI) the sensor apparatus (3) determines the resulting ventilation of the lungs and wherein the second sensor apparatus (30) determines the carbon dioxide supply and/or wherein the third sensor apparatus (40) determines the oxygen supply of the patient, wherein the control apparatus (21) stores the sensor measured values which are present during artificial respiration with the second pattern, and wherein the control apparatus (21) compares the sensor measured values which are present after the application of the first artificial respiration pattern with the sensor measured values which are present after the application of the second artificial respiration pattern and/or represents said values in comparative form on the display.

16. The artificial respiration device according to at least one of the preceding claims, **characterized in that** the control apparatus (21) monitors at least one sensor signal (2, 3, 30, 40) for errors, **in that** when the limit values of an instantaneous sensor signal (2, 3, 30, 40) are reached this instantaneous sensor signal is compared with a further instantaneous sensor signal (2, 3, 30, 40) in order to identify an error state.

## Revendications

1. Système respiratoire (10) comportant au moins un appareil respiratoire (1) avec au moins une source de gaz respirable commandable (100) et un dispositif de commande programmable (21) et au moins un dispositif de détection (2) pour la détermination d'une pression et/ou d'un flux du gaz respirable, dans lequel le dispositif de commande (21) active la source de gaz respiratoire pour la spécification d'un premier modèle respiratoire (45) (quant à la pression, au flux, au volume, à la fréquence), dans lequel le système respiratoire présente au moins deux autres dispositifs de détection (3, 30, 40), dans lequel un dispositif de détection (3) présente une pluralité de détecteurs individuels (3, 3', 3"...), lesquels sont conçus pour générer et/ou pour mesurer des potentiels électriques et conçus pour déterminer de façon non invasive l'impédance électrique (EI) des poumons d'un patient, dans lequel le deuxième dispositif de détection (30) est conçu pour déterminer de façon non invasive l'alimentation en dioxyde de carbone, dans lequel le troisième dispositif de détection (40) est conçu pour déterminer de façon non invasive l'alimentation en oxygène du patient, dans lequel le dispositif de commande (21) exploite les valeurs de mesure détectées du dispositif de détection d'EI (3) pour déterminer la ventilation actuelle (44) des poumons pendant l'assistance respiratoire selon le premier modèle respiratoire (45), dans lequel le dispositif de commande (21) exploite les valeurs de mesure détectées du deuxième dispositif de détection (30) pour déterminer l'alimentation en dioxyde de carbone actuelle (46) et dans lequel le dispositif de commande (21) exploite les valeurs de mesure détectées du troisième dispositif de détection (40) pour déterminer l'alimentation en oxygène actuelle (47), **caractérisé en ce que** le dispositif de commande (21) exploite les valeurs de mesure détectées du dispositif de détection d'EI (3) pour déterminer la ventilation résultante (54) des poumons pendant l'assistance respiratoire selon le deuxième modèle respiratoire (55), dans lequel le dispositif de commande (21) exploite les valeurs de mesure détectées du deuxième dispositif de détection (30) pour déterminer l'alimentation en dioxyde de carbone résultante (56) pendant l'assistance respiratoire selon le deuxième modèle respiratoire (55), dans lequel le dispositif de commande (21) exploite les valeurs de mesure détectées du troisième dispositif de détection (40) pour déterminer l'alimentation en oxygène résultante (57) pendant l'assistance respiratoire selon le deuxième modèle respiratoire (55) et dans lequel le dispositif de commande (21) compare la ventilation actuelle (44) avec la ventilation résultante (54), dans lequel le dispositif de commande (21) utilise les informations obtenues à l'aide des valeurs de mesure détectées concernant la ventilation des poumons et l'alimentation en oxygène et l'alimentation en dioxyde de carbone pour commander ou régler l'assistance respiratoire.

2. Système respiratoire selon la revendication 1, **caractérisé en ce que** le dispositif de commande (21) active la source de gaz respiratoire pour la spécification d'un premier modèle respiratoire (45) et enregistre les premières valeurs (de mesure) détectées résultantes d'au moins un dispositif de détection (2, 3, 30, 40), dans lequel le dispositif de commande (21) active ensuite la source de gaz respiratoire pour la spécification d'un deuxième modèle respiratoire (55) et enregistre les deuxièmes valeurs (de mesure) détectées résultantes d'au moins un dispositif de détection (2, 3, 30, 40) et compare les premières valeurs (de mesure) détectées avec les deuxièmes valeurs de mesure détectées.

3. Système respiratoire selon la revendication 1 ou 2, **caractérisé en ce que** le système respiratoire (10) présente au moins une mémoire (31) communiquant avec le dispositif de commande, dans laquelle sont enregistrées au moins des valeurs standard (62) et/ou des valeurs seuil (63) pour l'alimentation en dioxyde de carbone et pour l'alimentation en oxygène.

4. Système respiratoire selon la revendication 1, 2 ou 3, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque les valeurs de mesure détectées de l'alimentation en dioxyde de carbone actuelle (46) et/ou les valeurs de mesure détectées de l'alimentation en oxygène actuelle (47) s'écartent des valeurs standard (62) et/ou lorsque des valeurs seuil (62) sont au moins atteintes.

5. Système respiratoire selon l'une au moins des revendications précédentes, **caractérisé en ce que** des valeurs standard (72) et des valeurs seuil (73) sont enregistrées dans la mémoire (31) pour la ventilation (44) des poumons et le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque la ventilation actuelle (44) s'écarte des valeurs standard (72) et que des valeurs seuil (73) sont au moins atteintes.

6. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, de sorte que les valeurs de mesure détectées de l'alimentation en dioxyde de carbone et en oxygène résultantes se rapprochent au moins de valeurs seuil.

7. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque les valeurs de mesure détectées de la ventilation actuelle (44) et/ou les valeurs de mesure détectées de la pression partielle d'oxygène actuelle (47) s'écartent des valeurs standard (62) et que des valeurs seuil (63) sont au moins atteintes.

8. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque le dispositif de détection (3) identifie un signal de somme EIT (44') inchangé et une fréquence de modification EIT (44") élevée ou plus élevée, dans lequel le deuxième modèle respiratoire (55) présente une fréquence obligatoire plus élevée en comparaison avec le premier modèle respiratoire (45).

9. Système respiratoire selon l'une au moins des revendications précédentes, **caractérisé en ce que** le dispositif de commande (21) active 'la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque le dispositif de détection (3) présente un signal de somme EIT inchangé (44') en comparaison avec le premier modèle respiratoire (45) et un niveau plus faible pour l'alimentation en dioxyde de carbone, dans lequel le deuxième modèle respiratoire (55) présente une fréquence obligatoire plus élevée en comparaison avec le premier modèle respiratoire (45).

10. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque le dispositif de détection (3) présente un signal de somme EIT plus faible (44') en comparaison avec le premier modèle respiratoire (45), et un niveau plus faible pour l'alimentation en dioxyde de carbone, dans lequel le deuxième modèle respiratoire (55) présente une fréquence obligatoire plus élevée et/ou un volume tidal plus élevé et/ou une assistance de pression plus élevée, en comparaison avec le premier modèle respiratoire (45).

11. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque le dispositif de détection (3) présente un signal de somme EIT inchangé (44') et un niveau plus élevé pour l'alimentation en dioxyde de carbone, dans lequel le deuxième modèle respiratoire (55) présente une pression ou une évolution EPAP modifiée en comparaison avec le premier modèle respiratoire (45).

12. Système respiratoire selon l'une au moins des revendications précédentes, **caractérisé en ce que** le dispositif de commande (21) active la source de gaz respiratoire pour la spécification d'un deuxième modèle respiratoire (55), lequel se distingue du premier modèle respiratoire (45) quant à la pression et/ou au flux et/ou au volume et/ou à la fréquence, lorsque le dispositif de détection (3) présente un signal de somme EIT inchangé (44') en comparaison avec le premier modèle respiratoire (45), et un niveau plus élevé pour l'alimentation en dioxyde de carbone, dans lequel le deuxième modèle respiratoire (55) peut présenter une fréquence obligatoire plus élevée en comparaison avec le premier modèle respiratoire (45) lorsque la fréquence de modification EIT (44") est plus faible.

13. Système respiratoire selon l'une au moins des revendications précédentes, **caractérisé en ce que** le dispositif de commande (21) active la source de gaz respiratoire pour la spécification d'un deuxième modèle respiratoire (55), lorsque le dispositif de détection (3) présente un signal de somme EIT plus élevé (44') et/ou une fréquence de modification EIT plus faible, en comparaison avec le premier modèle respiratoire (45), et présente un niveau plus faible pour l'alimentation en oxygène, dans lequel le deuxième modèle respiratoire (55) peut présenter une fréquence plus élevée et/ou une augmentation du moins momentanée du temps d'inspiration et/ou une adaptation du PEEP et/ou un changement du modèle respiratoire, en comparaison avec le premier modèle respiratoire (45).

14. Système respiratoire selon l'une au moins des revendications précédentes, **caractérisé en ce que** le dispositif de commande (21) active la source de gaz respiratoire pour la spécification d'un premier PEEP (45) et enregistre les premières valeurs (de mesure) détectées résultantes d'au moins un dispositif de détection (2, 3, 30, 40), dans lequel le dispositif de commande (21) active ensuite la source de gaz respiratoire pour la spécification d'un deuxième PEEP (55) et enregistre les deuxièmes valeurs (de mesure) détectées résultantes d'au moins un dispositif de détection (2, 3, 30, 40), dans lequel le dispositif de commande (21) compare ensuite les premières valeurs (de mesure) détectées avec les deuxièmes valeurs de mesure détectées.

15. Système respiratoire selon l'une au moins des revendications précédentes, **caractérisé en ce que** le dispositif de commande (21) active la source de gaz respiratoire pour la spécification d'un premier modèle respiratoire (45), où la pression EPAP présente une première valeur ou une première évolution, dans lequel le dispositif de détection (3) détermine la ventilation résultante des poumons pour déterminer de façon non invasive l'impédance électrique (EI) et dans lequel le deuxième dispositif de détection (30) détermine l'alimentation en dioxyde de carbone et/ou dans lequel le troisième dispositif de détection (40) détermine l'alimentation en oxygène du patient, dans lequel le dispositif de commande (21) enregistre les valeurs de mesure détectées obtenues avec l'assistance respiratoire selon le premier modèle, dans lequel le dispositif de commande (21) active la source de gaz respiratoire pour la spécification d'un deuxième modèle respiratoire (55), où la pression EPAP présente une deuxième valeur ou une deuxième évolution, dans lequel le dispositif de détection (3) détermine la ventilation résultante des poumons pour déterminer de façon non invasive l'impédance électrique (EI) et dans lequel le deuxième dispositif de détection (30) détermine l'alimentation en dioxyde de carbone et/ou dans lequel le troisième dispositif de détection (40) détermine l'alimentation en oxygène du patient, dans lequel le dispositif de commande (21) enregistre les valeurs de mesure détectées obtenues avec l'assistance respiratoire selon le deuxième modèle, et dans lequel le dispositif de commande (21) compare les valeurs dè mesure détectées obtenues après application du premier modèle respiratoire avec les valeurs de mesure détectées obtenues après application du deuxième modèle respiratoire et/ou représente celles-ci sur un écran.

16. Système respiratoire selon l'une au moins des revendications précédentes, **caractérisé en ce que** le dispositif de commande (21) surveille au moins un signal de détecteur (2, 3, 30, 40) à la recherche d'anomalies, par comparaison d'un signal de détecteur actuel (2, 3, 30, 40) atteignant des valeurs seuil avec d'autres signaux de détecteur actuels (2, 3, 30, 40) afin de détecter la présence d'anomalies.
